(19) Europäisches Patentamt
European Patent Office
Office européen des brevets

(11) Veröffentlichungsnummer: **0 337 232 A1**

# (12) EUROPÄISCHE PATENTANMELDUNG

(21) Anmeldenummer: **89105800.0**

(22) Anmeldetag: **03.04.89**

(51) Int. Cl.4: **C07D 487/04 , A01N 43/90 , //(C07D487/04,251:00,249:00), (C07D487/04,249:00,239:00)**

(30) Priorität: **14.04.88 DE 3812350**

(43) Veröffentlichungstag der Anmeldung: **18.10.89 Patentblatt 89/42**

(84) Benannte Vertragsstaaten: **BE CH DE ES FR GB IT LI NL SE**

(71) Anmelder: **BAYER AG**
**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Santel, Hans-Joachim, Dr.**
**Gruenstrasse 9 a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6 a**
**D-4000 Duesseldorf 31(DE)**

(54) **Triazoloazine.**

(57) Die Erfindung betrifft neue Triazoloazine der allgemeinen Formel I

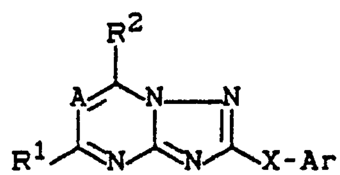

(I)

in welcher
A für Stickstoff oder für einen Rest C-$R^3$ steht,
$R^1$, $R^2$ und $R^3$ unabhängig voneinander jeweils für Wasserstoff, Hydroxy, Halogen, für gegebenenfalls substituiertes Alkyl, für Alkoxy, Alkylthio oder für gegebenenfalls substituiertes Aryl stehen oder entweder $R^1$ und $R^3$ gemeinsam oder $R^3$ und $R^2$ gemeinsam für einen ankondensierten gegebenenfalls substituierten Carbo- oder Heterocyclus stehen,
X für Sauerstoff, Schwefel, eine Sulfinylgruppe oder eine Sulfonylgruppe steht und
Ar für jeweils gegebenenfalls substituiertes Aryl oder Heteroaryl steht,
Verfahren zu ihrer Herstellung und ihre Verwendung als Herbizide.

EP 0 337 232 A1

## Triazoloazine

Die Erfindung betrifft neue Triazoloazine, mehrere Verfahren zu ihrer Herstellung und ihre Verwendung als Herbizide.

Es ist bekannt, daß bestimmte Triazolopyrimidine, wie beispielsweise die Verbindung 5,7-Dimethyl-N-(2-methoxycarbonylphenyl)-1,2,4-triazolo[1,5-a]pyrimidin-2-sulfonamid, herbizide Eigenschaften besitzen (vgl. z.B. EP 142 152).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Triazoloazine der allgemeinen Formel (I),

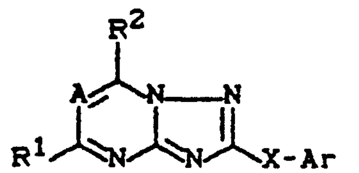

(I)

in welcher
A für Stickstoff oder für einen Rest C-$R^3$ steht,
$R^1$, $R^2$ und $R^3$ unabhängig voneinander jeweils für Wasserstoff, Hydroxy, Halogen, für gegebenenfalls substituiertes Alkyl, für Alkoxy, Alkylthio oder für gegebenenfalls substituiertes Aryl stehen oder entweder $R^1$ und $R^3$ gemeinsam oder $R^3$ und $R^2$ gemeinsam für einen ankondensierten gegebenenfalls substituierten Carbo- oder Heterocyclus stehen,
X für Sauerstoff, Schwefel, eine Sulfinylgruppe oder eine Sulfonylgruppe steht und
Ar für jeweils gegebenenfalls substituiertes Aryl oder Heteroaryl steht,
gefunden.

Weiterhin wurde gefunden, daß man die neuen Triazoloazine der allgemeinen Formel (I),

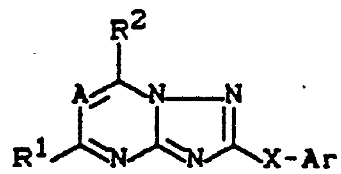

(I)

in welcher
A für Stickstoff oder für einen Rest C-$R^3$ steht,
$R^1$, $R^2$ und $R^3$ unabhängig voneinander jeweils für Wasserstoff, Hydroxy, Halogen, für gegebenenfalls substituiertes Alkyl, für Alkoxy, Alkylthio oder für gegebenenfalls substituiertes Aryl stehen oder entweder $R^1$ und $R^3$ gemeinsam oder $R^3$ und $R^2$ gemeinsam für einen ankondensierten gegebenenfalls substituierten Carbo- oder Heterocyclus stehen,
X für Sauerstoff, Schwefel, eine Sulfinylgruppe oder eine Sulfonylgruppe steht und
Ar für jeweils gegebenenfalls substituiertes Aryl oder Heteroaryl steht,
nach einem der im Folgenden beschriebenen Verfahren erhält:

(a) Man erhält Triazoloazine der Formel (Ia),

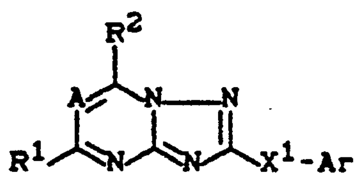

(Ia)

in welcher
$X^1$ für Sauerstoff oder Schwefel steht und

$R^1$, $R^2$, A und Ar die oben angegebene Bedeutung haben,
wenn man Triazoloazin-Derivate der Formel (II),

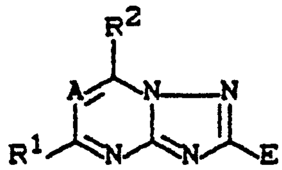

(II)

in welcher
E für Halogen, für Alkylthio oder für Alkylsulfonyl steht und
$R^1$, $R^2$ und A die oben angegebene Bedeutung haben,
mit aromatischen Alkoholen oder Thiolen der Formel (III),
$Ar-X^1-H$ (III)
in welcher
$X^1$ und Ar die oben angegebene Bedeutung haben,
gegebenenfalls in Gegenwart eines Verdünnungsmittels und gegebenenfalls in Gegenwart eines Reaktionshilfsmittels umsetzt;

(b) man erhält Triazoloazine der Formel (Ib),

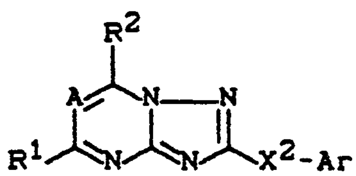

(Ib)

in welcher
$X^2$ für eine Sulfinylgruppe oder eine Sulfonylgruppe steht und
$R^1$, $R^2$, A und Ar die oben angegebene Bedeutung haben,
wenn man die mit Hilfe des Verfahrens (a) erhältlichen Triazoloazine der Formel (Ic),

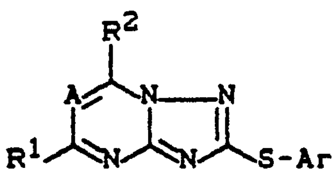

(Ic)

in welcher
$R^1$, $R^2$, A und Ar die oben angegebene Bedeutung haben,
mit einem Oxidationsmittel gegebenenfalls in Gegenwart eines Verdünnungsmittels und gegebenenfalls in Gegenwart eines Katalysators umsetzt.

Schließlich wurde gefunden, daß die neuen Triazoloazine der allgemeinen Formel (I) gute herbizide Eigenschaften besitzen.

Überraschenderweise zeigen die erfindungsgemäßen Triazoloazine der allgemeinen Formel (I) eine erheblich bessere herbizide Wirksamkeit als die aus dem Stand der Technik bekannten Triazolopyrimidine, wie beispielsweise die Verbindung 5,7-Dimethyl-N-(2-methoxycarbonylphenyl)-1,2,4-triazolo[1,5-a]pyrimidin-2-sulfonamid, welche chemisch und wirkungsmäßig naheliegende Verbindungen sind.

Die erfindungsgemäßen Triazoloazine sind durch die Formel (I) allgemein definiert. Bevorzugt sind Verbindungen der Formel (I), bei welchen
A für Stickstoff oder für einen Rest $C-R^3$ steht,
$R^1$, $R^2$ und $R^3$ unabhängig voneinander jeweils für Wasserstoff, Hydroxy, Fluor, Chlor, Brom, Iod, für jeweils geradkettiges oder verzweigtes Alkyl, Alkoxy oder Alkylthio mit jeweils 1 bis 6 Kohlenstoffatomen, für jeweils geradkettiges oder verzweigtes Alkoxyalkyl oder Alkylthioalkyl mit jeweils 1 bis 6 Kohlenstoffatomen in den einzelnen Alkylteilen, für geradkettiges oder verzweigtes Halogenalkyl mit 1 bis 4 Kohlenstoffatomen

und 1 bis 9 gleichen oder verschiedenen Halogenatomen oder für gegebenenfalls einfach oder mehrfach, gleich oder verschieden substituiertes Aryl mit 6 bis 10 Kohlenstoffatomen stehen, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder jeweils geradkettiges oder verzweigtes Alkoxycarbonyl oder Alkoximinoalkyl mit jeweils 1 bis 4 Kohlenstoffatomen in den einzelnen Alkylteilen; oder entweder $R^1$ und $R^3$ gemeinsam oder $R^3$ und $R^2$ gemeinsam für einen ankondensierten gegebenenfalls einfach oder mehrfach, gleich oder verschieden substituierten, gesättigten oder ungesättigten Carbo- oder Heterocyclus stehen, wobei im Fall des Heterocyclus 1 bis 3 gleiche oder verschiedene Heteroatome - insbesondere Stickstoff, Sauerstoff oder Schwefel - enthalten sein können und wobei als Substituenten jeweils die oben genannten Arylsubstituenten infrage kommen,

X für Sauerstoff, Schwefel, eine Sulfinylgruppe oder eine Sulfonylgruppe steht und

Ar für gegebenenfalls einfach oder mehrfach, gleich oder verschieden substituiertes Aryl mit 6 bis 10 Kohlenstoffatomen oder für einen gegebenenfalls einfach oder mehrfach, gleich oder verschieden substituierten und/oder benzannellierten 5- bis 7-gliedrigen Heterocyclus mit 1 bis 3 Heteroatomen (insbesondere Stickstoff, Sauerstoff und/oder Schwefel) steht, wobei als Substituenten jeweils in Frage kommen: Fluor, Chlor, Brom, Iod, Cyano, Nitro, Hydroxy, jeweils geradkettiges oder verzweigtes Alkyl, Alkoxy, Alkylthio, Alkylcarbonyl, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl oder Halogenalkylcarbonyl mit jeweils 1 bis 6 Kohlenstoffatomen und 1 bis 9 gleichen oder verschiedenen Halogenatomen, Phenyl, Phenoxy, Phenylthio, Phenylcarbonyl, Hydroxycarbonyl, jeweils geradkettiges oder verzweigtes Alkoxycarbonyl, Alkenyloxycarbonyl oder Alkoxyalkoxycarbonyl mit jeweils 1 bis 6 Kohlenstoffatomen in den einzelnen Alkylteilen bzw. 3 bis 6 Kohlenstoffatomen im Alkenylteil, sowie Hydroximinoalkyl oder geradkettiges oder verzweigtes Alkoximinoalkyl mit jeweils 1 bis 4 Kohlenstoffatomen in den einzelnen Alkylteilen.

Besonders bevorzugt sind Verbindungen der allgemeinen Formel (I), bei welchen

A für Stickstoff oder für einen Rest C-$R^3$ steht,

$R^1$, $R^2$ und $R^3$ unabhängig voneinander jeweils für Wasserstoff, Hydroxy, Fluor, Chlor, Brom, für Methyl, Ethyl, n- oder i-Propyl, n-, i-, s- oder t-Butyl, für Chlormethyl, Dichlormethyl, Trichlormethyl, Fluormethyl, Difluormethyl, Trifluormethyl, Dichlorfluormethyl, Chlordifluormethyl oder Fluorchlormethyl, für Methoxymethyl, Ethoxymethyl, n- oder i-Propoxymethyl, Methylthiomethyl, Ethylthiomethyl, n-oder i-Propylthiomethyl, für Methoxy, Ethoxy, n- oder i-Propoxy, für Methylthio, Ethylthio, n- oder i-Propylthio oder für Phenyl stehen,

X für Sauerstoff, Schwefel, eine Sulfinylgruppe oder eine Sulfonylgruppe steht und

Ar für jeweils gegebenenfalls ein- bis dreifach, gleich oder verschieden substituiertes Phenyl oder Naphthyl oder für einen gegebenenfalls ein- bis dreifach gleich oder verschieden substituierten und/oder benzannellierten Heterocyclus der Formel

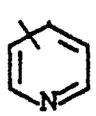 ; 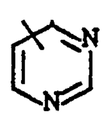 ; 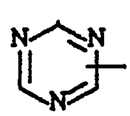 ; 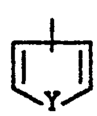 ; 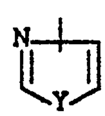 ;

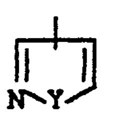 ; 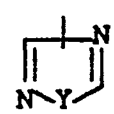 oder 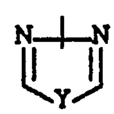 steht,

wobei Y jeweils für Sauerstoff, Schwefel, eine NH-Gruppe oder eine N-$CH_3$-Gruppe steht und wobei als Phenyl-, Naphthyl- und Heterocyclyl-Substituenten jeweils in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, n- oder i-Propoxy, Methylthio, Ethylthio, Acetyl, Propionyl, Methylsulfinyl, Methylsulfonyl, Trifluormethyl, Trifluormethoxy, Trifluormethylthio, Trifluormethylsulfinyl, Trifluormethylsulfonyl, Chloracetyl, Dichloracetyl, Trifluoracetyl, Phenyl, Phenoxy, Phenylthio, Benzoyl, Hydroxycarbonyl, Methoxycarbonyl, Ethoxycarbonyl, n- oder i-Propoxycarbonyl, Allyloxycarbonyl, Methoxymethoxycarbonyl, Ethoxyethoxycarbonyl, Hydroximinomethyl, Methoximinomethyl, Methoximinoethyl und Ethoximinioethyl.

Ganz besonders bevorzugt sind Verbindungen der allgemeinen Formel (I), bei welchen

A für Stickstoff oder für eine CH-Gruppe steht,

$R^1$ und $R^2$ unabhängig voneinander für Wasserstoff, für Methyl, Ethyl, n- oder i-Propyl, n-, i-, s-oder t-Butyl, für Methoxymethyl, Ethoxymethyl, n- oder i-Propoxymethyl, für Methylthiomethyl, für Trifluormethyl, Trichlormethyl, für Dichlorfluormethyl, Chlordifluormethyl, für Methoxy, Ethoxy, n- oder i-Propoxy, Methylthio oder Ethylthio stehen,
X für Sauerstoff, Schwefel, eine Sulfinylgruppe oder eine Sulfonylgruppe steht und
Ar für jeweils gegebenenfalls ein- bis dreifach, gleich oder verschieden substituiertes Phenyl, α-Naphthyl oder ß-Naphthyl steht oder für einen gegebenenfalls ein- bis dreifach gleich oder verschieden substituierten und/oder benzannellierten Heterocyclus der Formel

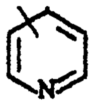 ; 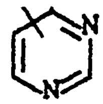 ; 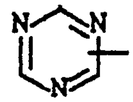 ; 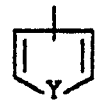 ; 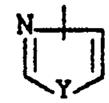 oder

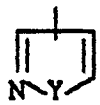 steht,

wobei Y jeweils für Sauerstoff, Schwefel, eine NH-Gruppe oder eine N-$CH_3$-Gruppe steht und wobei als Phenyl-, Naphthyl- und Heterocyclyl-Substituenten jeweils infrage kommen: Fluor, Chlor, Brom, Iod, Cyano, Nitro, Hydroxy, Methyl, Ethyl, Methoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

Insbesondere bevorzugt sind Verbindungen der Formel (I), bei welchen
A für eine CH-Gruppe steht,
$R^1$ für Methyl, Methoxy, Methoxymethyl oder Trifluormethyl steht,
$R^2$ für Wasserstoff, Methyl, Methoxy, Methoxymethyl oder Trifluormethyl steht,
X für Sauerstoff, Schwefel oder eine Sulfonylgruppe steht und
Ar für jeweils gegebenenfalls ein- bis dreifach, gleich oder verschieden substituiertes Phenyl, Pyridyl oder Pyrimidyl steht, wobei als Substituenten jeweils in Frage kommen: Fluor, Chlor, Brom, Cyano, Nitro, Hydroxy, Methyl, Ethyl, t-Butyl, Methoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

Im einzelnen seien außer den bei den Herstellungsbeispielen genannten Verbindungen die folgenden Triazoloazine der allgemeinen Formel (I) genannt:

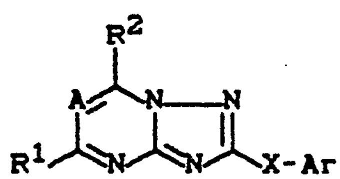 (I)

Tabelle 1

| $R^1$ | $R^2$ | A | X | Ar |
|---|---|---|---|---|
| $CH_3$ | $CH_3$ | CH | O | 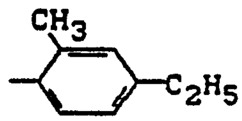 |
| $CH_3$ | $CH_3$ | CH | O | 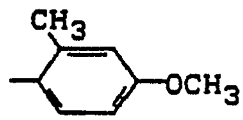 |
| $CH_3$ | $CH_3$ | CH | O | 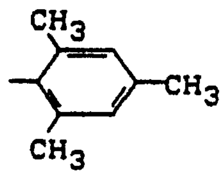 |
| $CH_3$ | $CH_3$ | CH | O | 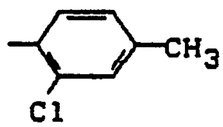 |
| $CH_3$ | $CH_3$ | CH | O | 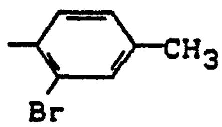 |
| $CH_3$ | $CH_3$ | CH | O | 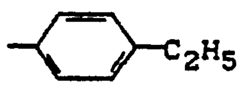 |
| $CH_3$ | $CH_3$ | CH | O | 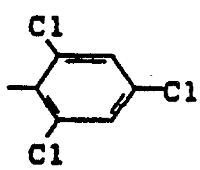 |
| $CH_3$ | $CH_3$ | CH | O | 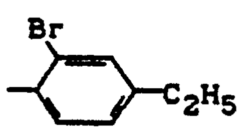 |

<u>Tabelle 1</u> (Fortsetzung)

| $R^1$ | $R^2$ | A | X | Ar |
|---|---|---|---|---|
| $CH_3$ | $CH_3$ | CH | O | 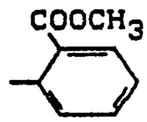 |
| $CH_3$ | $CH_3$ | CH | O | 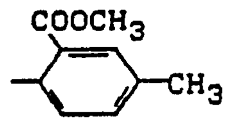 |
| $CH_3$ | $CH_3$ | CH | O | 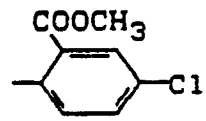 |
| $CH_3$ | $CH_3$ | CH | S | 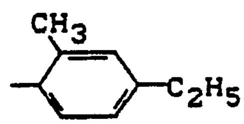 |
| $CH_3$ | $CH_3$ | CH | S | 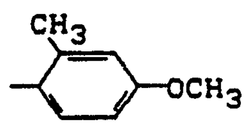 |
| $CH_3$ | $CH_3$ | CH | S | 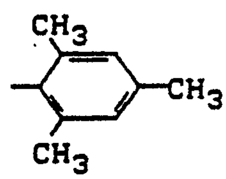 |
| $CH_3$ | $CH_3$ | CH | S | 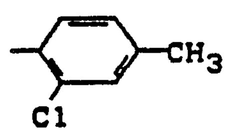 |
| $CH_3$ | $CH_3$ | CH | S | 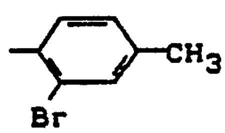 |
| $CH_3$ | $CH_3$ | CH | S | 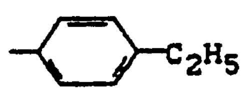 |

Tabelle 1 (Fortsetzung)

| $R^1$ | $R^2$ | A | X | Ar |
|---|---|---|---|---|
| $CH_3$ | $CH_3$ | CH | S | 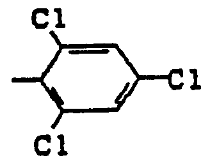 |
| $CH_3$ | $CH_3$ | CH | S | 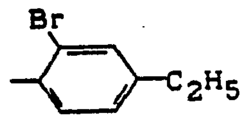 |
| $CH_3$ | $CH_3$ | CH | S | 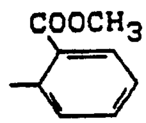 |
| $CH_3$ | $CH_3$ | CH | S | 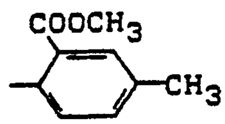 |
| $CH_3$ | $CH_3$ | CH | S | 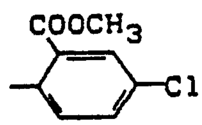 |
| $CH_3$ | $CH_3$ | CH | 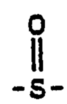 | 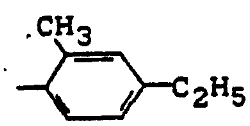 |
| $CH_3$ | $CH_3$ | CH | 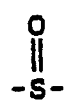 | 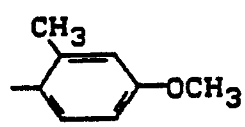 |
| $CH_3$ | $CH_3$ | CH | 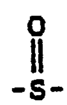 | 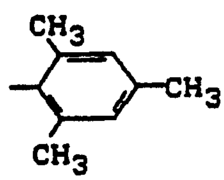 |

Tabelle 1 (Fortsetzung)

| $R^1$ | $R^2$ | A | X | Ar |
|---|---|---|---|---|
| $CH_3$ | $CH_3$ | CH | 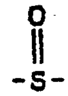 | 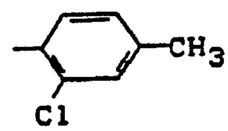 |
| $CH_3$ | $CH_3$ | CH | 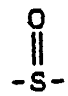 | 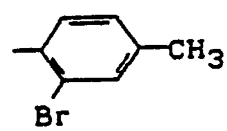 |
| $CH_3$ | $CH_3$ | CH | 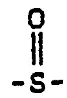 | 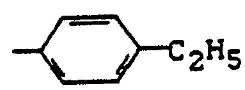 |
| $CH_3$ | $CH_3$ | CH | 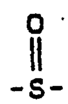 | 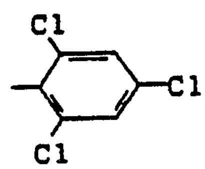 |
| $CH_3$ | $CH_3$ | CH | 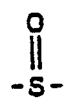 | 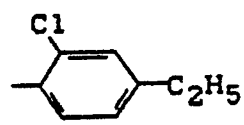 |
| $CH_3$ | $CH_3$ | CH | 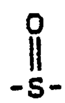 | 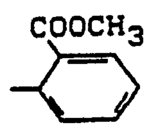 |
| $CH_3$ | $CH_3$ | CH | 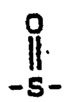 | 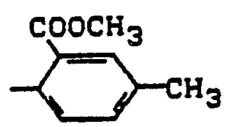 |
| $CH_3$ | $CH_3$ | CH | 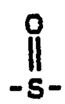 | 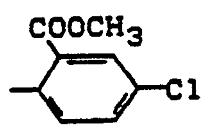 |

Tabelle 1 (Fortsetzung)

| $R^1$ | $R^2$ | A | X | Ar |
|---|---|---|---|---|
| $CH_3$ | $CH_3$ | CH | $-SO_2-$ | 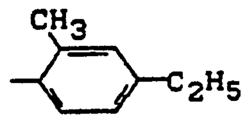 |
| $CH_3$ | $CH_3$ | CH | $-SO_2-$ | 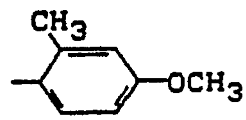 |
| $CH_3$ | $CH_3$ | CH | $-SO_2-$ | 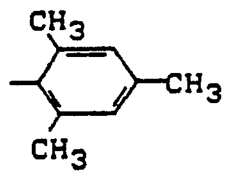 |
| $CH_3$ | $CH_3$ | CH | $-SO_2-$ | 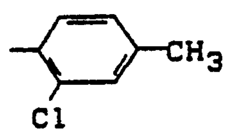 |
| $CH_3$ | $CH_3$ | CH | $-SO_2-$ | 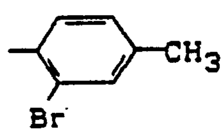 |
| $CH_3$ | $CH_3$ | CH | $-SO_2-$ | 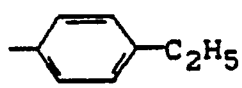 |
| $CH_3$ | $CH_3$ | CH | $-SO_2-$ | 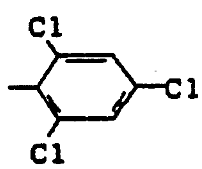 |
| $CH_3$ | $CH_3$ | CH | $-SO_2-$ | 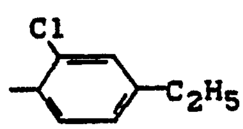 |

**Tabelle 1** (Fortsetzung)

| $R^1$ | $R^2$ | A | X | Ar |
|---|---|---|---|---|
| $CH_3$ | $CH_3$ | CH | $-SO_2-$ | 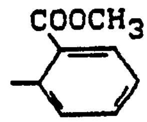 |
| $CH_3$ | $CH_3$ | CH | $-SO_2-$ | 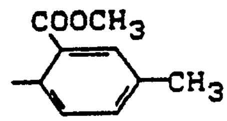 |
| $CH_3$ | $CH_3$ | CH | $-SO_2-$ | 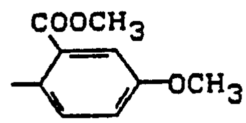 |
| $CH_3$ | $CH_3$ | N | O | 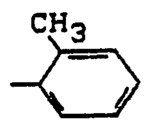 |
| $CH_3$ | $CH_3$ | N | O | 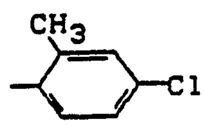 |
| $CH_3$ | $CH_3$ | N | O | 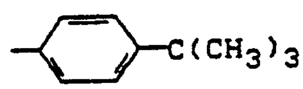 |
| $CH_3$ | $CH_3$ | N | O | 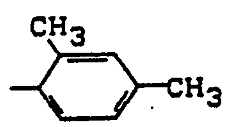 |
| $CH_3$ | $CH_3$ | N | O | 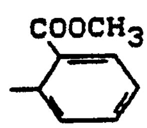 |
| $CH_3$ | $CH_3$ | N | S | 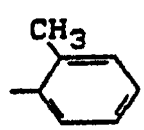 |

Tabelle 1 (Fortsetzung)

| $R^1$ | $R^2$ | A | X | Ar |
|---|---|---|---|---|
| $CH_3$ | $CH_3$ | N | S | 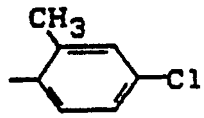 |
| $CH_3$ | $CH_3$ | N | S | 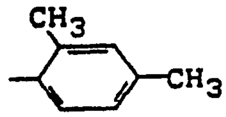 |
| $CH_3$ | $CH_3$ | N | S | 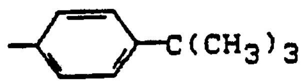 |
| $CH_3$ | $CH_3$ | N | S | 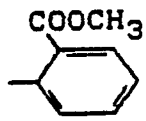 |
| $CH_3$ | $CH_3$ | N | $-SO_2-$ | 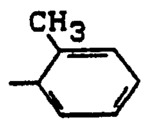 |
| $CH_3$ | $CH_3$ | N | $-SO_2-$ | 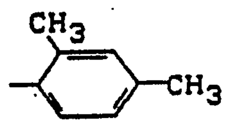 |
| $CH_3$ | $CH_3$ | N | $-SO_2-$ | 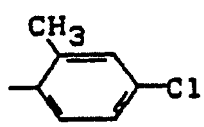 |
| $CH_3$ | $CH_3$ | N | $-SO_2-$ | 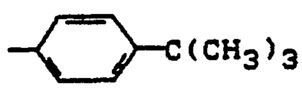 |
| $CH_3$ | $CH_3$ | N | $-SO_2-$ | 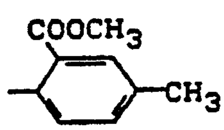 |

**Tabelle 1** (Fortsetzung)

| $R^1$ | $R^2$ | A | X | Ar |
|---|---|---|---|---|
| $CH_3$ | $CH_3$ | N | $-SO_2-$ | 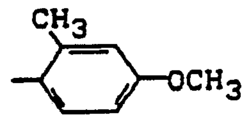 |
| $CH_3$ | $CH_3$ | N | 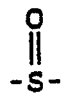 | 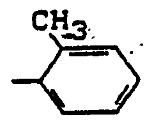 |
| $CH_3$ | $CH_3$ | N | 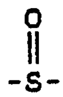 | 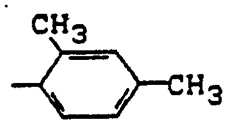 |
| $CH_3$ | $CH_3$ | N | 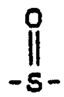 | 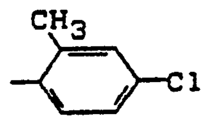 |
| $CH_3$ | $CH_3$ | N | 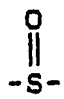 | 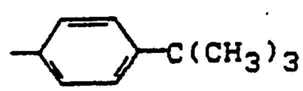 |
| $CH_3$ | $CH_3$ | N | 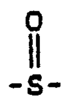 | 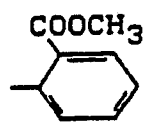 |
| $CH_3$ | $OCH_3$ | CH | O | 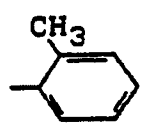 |
| $CH_3$ | $OCH_3$ | CH | O | 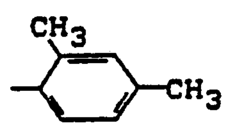 |
| $CH_3$ | $OCH_3$ | CH | O | 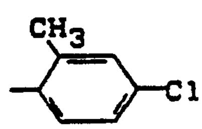 |

Tabelle 1 (Fortsetzung)

| $R^1$ | $R^2$ | A | X | Ar |
|---|---|---|---|---|
| $CH_3$ | $OCH_3$ | CH | O | 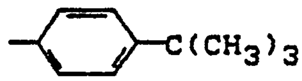 |
| $CH_3$ | $OCH_3$ | CH | O | 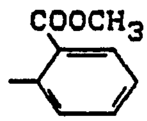 |
| $CH_3$ | $OCH_3$ | CH | O | 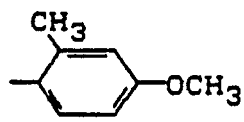 |
| $CH_3$ | $OCH_3$ | CH | S | 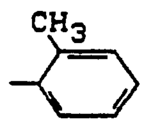 |
| $CH_3$ | $OCH_3$ | CH | S | 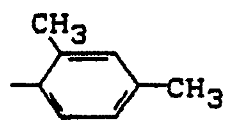 |
| $CH_3$ | $OCH_3$ | CH | S | 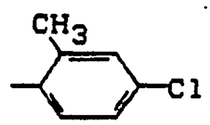 |
| $CH_3$ | $OCH_3$ | CH | S | 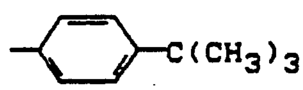 |
| $CH_3$ | $OCH_3$ | CH | S | 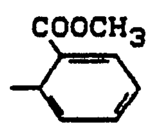 |
| $CH_3$ | $OCH_3$ | CH | S | 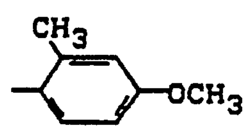 |

**Tabelle 1** (Fortsetzung)

| $R^1$ | $R^2$ | A | X | Ar |
|---|---|---|---|---|
| $CH_3$ | $OCH_3$ | CH | 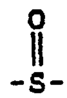 | 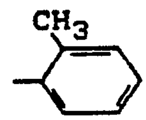 |
| $CH_3$ | $OCH_3$ | CH | 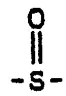 | 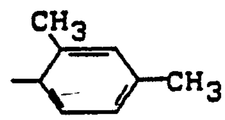 |
| $CH_3$ | $OCH_3$ | CH | 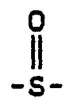 | 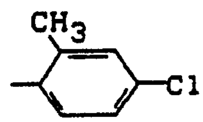 |
| $CH_3$ | $OCH_3$ | CH | 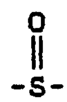 | 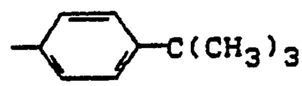 |
| $CH_3$ | $OCH_3$ | CH | 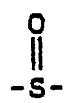 | 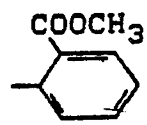 |
| $CH_3$ | $OCH_3$ | CH | $-SO_2-$ | 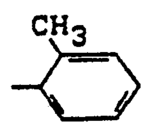 |
| $CH_3$ | $OCH_3$ | CH | $-SO_2-$ | 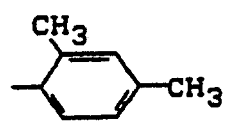 |
| $CH_3$ | $OCH_3$ | CH | $-SO_2-$ | 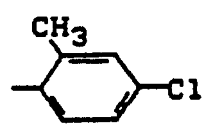 |
| $CH_3$ | $OCH_3$ | CH | $-SO_2-$ | 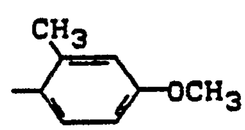 |

Tabelle 1 (Fortsetzung)

| $R^1$ | $R^2$ | A | X | Ar |
|---|---|---|---|---|
| $CH_3$ | $OCH_3$ | CH | $-SO_2-$ | 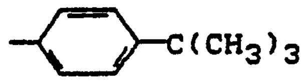 |
| $CH_3$ | $OCH_3$ | CH | $-SO_2-$ | 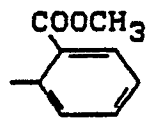 |
| $OCH_3$ | $OCH_3$ | CH | O | 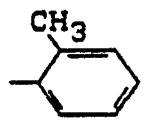 |
| $OCH_3$ | $OCH_3$ | CH | O | 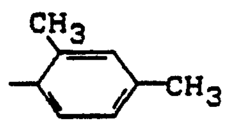 |
| $OCH_3$ | $OCH_3$ | CH | O | 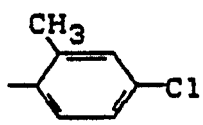 |
| $OCH_3$ | $OCH_3$ | CH | O | 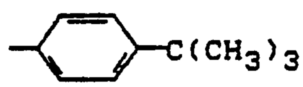 |
| $OCH_3$ | $OCH_3$ | CH | O | 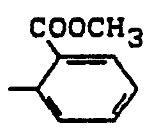 |
| $OCH_3$ | $OCH_3$ | CH | S | 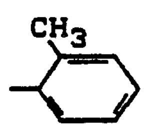 |
| $OCH_3$ | $OCH_3$ | CH | S | 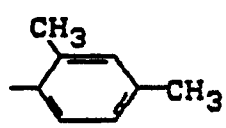 |

<u>Tabelle 1</u> (Fortsetzung)

| $R^1$ | $R^2$ | A | X | Ar |
|---|---|---|---|---|
| $OCH_3$ | $OCH_3$ | CH | S | 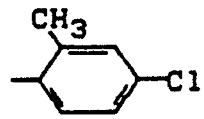 |
| $OCH_3$ | $OCH_3$ | CH | S | 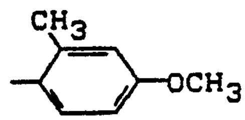 |
| $OCH_3$ | $OCH_3$ | CH | S | 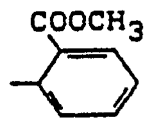 |
| $OCH_3$ | $OCH_3$ | CH | S | 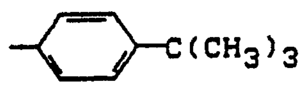 |
| $OCH_3$ | $OCH_3$ | CH | $-\overset{\overset{O}{\|}}{S}-$ | 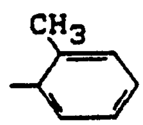 |
| $OCH_3$ | $OCH_3$ | CH | $-\overset{\overset{O}{\|}}{S}-$ | 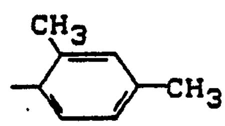 |
| $OCH_3$ | $OCH_3$ | CH | $-\overset{\overset{O}{\|}}{S}-$ | 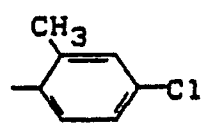 |
| $OCH_3$ | $OCH_3$ | CH | $-\overset{\overset{O}{\|}}{S}-$ | 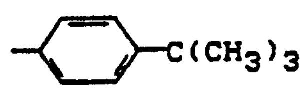 |
| $OCH_3$ | $OCH_3$ | CH | $-\overset{\overset{O}{\|}}{S}-$ | 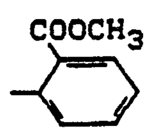 |

<u>Tabelle 1</u> (Fortsetzung)

| $R^1$ | $R^2$ | A | X | Ar |
|---|---|---|---|---|
| $OCH_3$ | $OCH_3$ | CH | $-SO_2-$ | 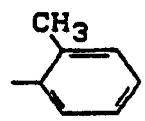 |
| $OCH_3$ | $OCH_3$ | CH | $-SO_2-$ | 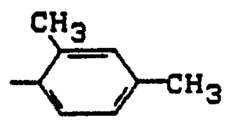 |
| $OCH_3$ | $OCH_3$ | CH | $-SO_2-$ | 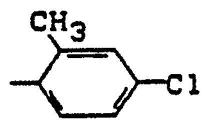 |
| $OCH_3$ | $OCH_3$ | CH | $-SO_2-$ | 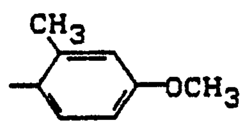 |
| $OCH_3$ | $OCH_3$ | CH | $-SO_2-$ | 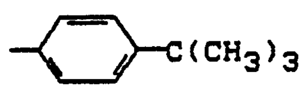 |
| $OCH_3$ | $OCH_3$ | CH | $-SO_2-$ | 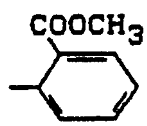 |
| $CH_3$ | $CH_3$ | CH | O | 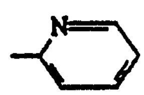 |
| $CH_3$ | $CH_3$ | CH | O | 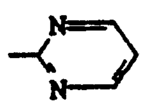 |
| $CH_3$ | $CH_3$ | CH | O | 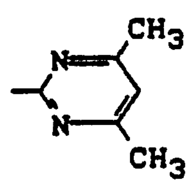 |

Tabelle 1 (Fortsetzung)

| $R^1$ | $R^2$ | A | X | Ar |
|---|---|---|---|---|
| $CH_3$ | $CH_3$ | CH | O | 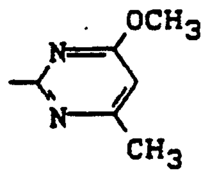 |
| $CH_3$ | $CH_3$ | CH | O | 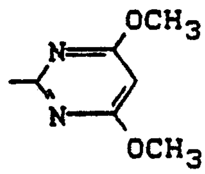 |
| $CH_3$ | $CH_3$ | CH | -S- | 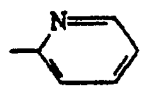 |
| $CH_3$ | $CH_3$ | CH | -S- | 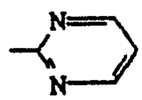 |
| $CH_3$ | $CH_3$ | CH | -S- | 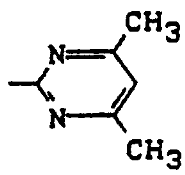 |
| $CH_3$ | $CH_3$ | CH | -S- | 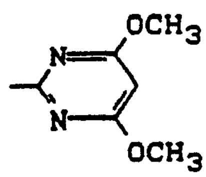 |
| $CH_3$ | $CH_3$ | CH | 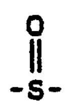 | 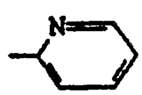 |
| $CH_3$ | $CH_3$ | CH | 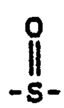 | 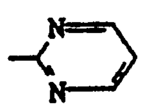 |

Tabelle 1 (Fortsetzung)

| $R^1$ | $R^2$ | A | X | Ar |
|---|---|---|---|---|
| $CH_3$ | $CH_3$ | CH | 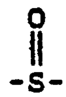 | 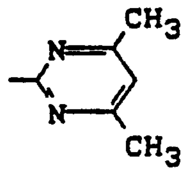 |
| $CH_3$ | $CH_3$ | CH | $-SO_2-$ | 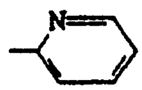 |
| $CH_3$ | $CH_3$ | CH | $-SO_2-$ | 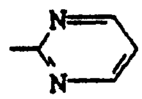 |
| $CH_3$ | $CH_3$ | CH | $-SO_2-$ | 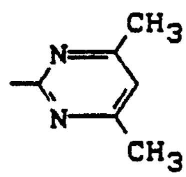 |
| $CH_3$ | $CH_3$ | CH | $-SO_2-$ | 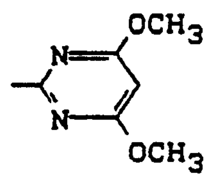 |

Verwendet man beispielsweise 2-Chlor-5,7-dimethyl-1,2,4-triazolo[1,5-a]pyrimidin und 2-Chlorthiophenol als Ausgangsstoffe, so läßt sich der Reaktionsablauf des erfindungsgemäßen Verfahrens (a) durch das folgende Formelschema darstellen:

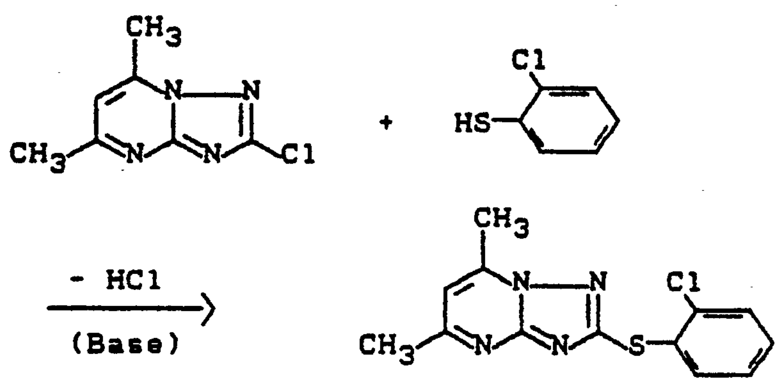


Verwendet man beispielsweise 2-(2-Chlorphenylthio)-5,7-dimethyl-1,2,4-triazolo[1,5-a]pyrimidin als Ausgangsverbindung und 3-Chlorperbenzoesäure als Oxidationsmittel, so läßt sich der Reaktionsablauf des erfindungsgemäßen Verfahrens (b) durch das folgende Formelschema darstellen:

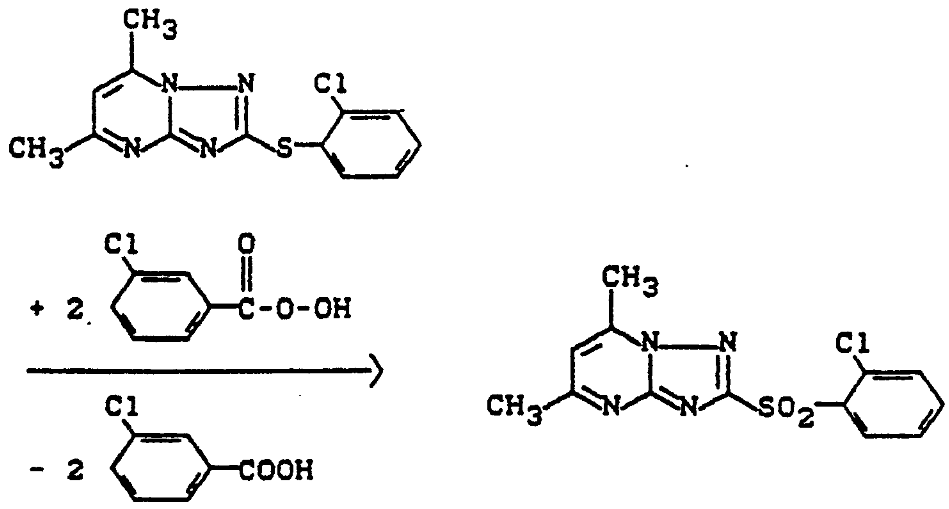

Die zur Durchführung des erfindungsgemäßen Verfahrens (a) als Ausgangsstoffe benötigten Triazoloazin-Derivate sind durch die Formel (II) allgemein definiert. In dieser Formel (II) stehen $R^1$, $R^2$ und A vorzugsweise für diejenigen Reste, die bereits im Zusammenhang mit der Beschreibung der erfindungsgemäßen Stoffe der Formel (I) als bevorzugt für diese Substituenten genannt wurden.

E steht in Formel (II) vorzugsweise für Chlor, Brom oder Iod, insbesondere für Chlor oder Brom oder für jeweils geradkettiges oder verzweigtes Alkylthio oder Alkylsulfonyl mit jeweils 1 bis 4 Kohlenstoffatomen.

Die Triazoloazin-Derivate der Formel (II) sind bekannt oder erhältlich in Analogie zu bekannten Verfahren (vgl. z.B. J. Heterocycl. Chem. 20, 735-751 [1983]; J. chem. Soc. C 1966, 2031-2038; Jp 55/113 043; J. Prakt. Chem. 314; 515-524 [1972]; DE 23 27 133; US 40 36 840; J. chem. Soc. Perkin Trans. I 1979, 3085-3094; Austral. J. Chem. 32, 2727-2732 [1979]; J. Photographic Sci. 28, 121-127 [1980]; Synthesis 1983, 44-47; EP 197 895).

Die zur Durchführung des erfindungsgemäßen Verfahrens (a) weiterhin als Ausgangsstoffe benötigten aromatischen Alkohole oder Thiole sind durch die Formel (III) allgemein definiert. In dieser Formel (III) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

$X^1$ steht in Formel (III) vorzugsweise für Sauerstoff oder Schwefel.

Die aromatischen Alkohole oder Thiole der Formel (III) sind allgemein bekannte Verbindungen der organischen Chemie.

Die zur Durchführung des erfindungsgemäßen Verfahrens (b) als Ausgangsstoffe benötigten Triazoloazine sind durch die Formel (Ic) allgemein definiert. In dieser Formel (Ic) stehen $R^1$, $R^2$, A und Ar vorzugsweise für diejenigen Reste, die bereits im Zuzammenhang mit der Beschreibung der erfindungsgemäßen Stoffe der Formel (I) als bevorzugt für diese Substituenten genannt wurden.

Die Triazoloazine der Formel (Ic) sind erfindungsgemäße Verbindungen und erhältlich mit Hilfe des erfindungsgemäßen Verfahrens (a).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethyl phosphorsäuretriamid, Ester, wie Essigsäureethylester oder Sulfoxide, wie Dimethylsulfoxid oder Sulfone, wie Sulfolan.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C

und 250 °C, vorzugsweise bei Temperaturen zwischen 80 °C und 230 °C.

Zur Durchführung des erfindungsgemäßen Verfahrens (a) setzt man pro Mol an Triazoloazin-Derivat der Formel (II) im allgemeinen 0.8 bis 2.5 Mol, vorzugsweise 1.0 bis 1.5 Mol an aromatischem Alkohol oder Thiol der Formel (III) und gegebenenfalls 0.8 bis 2.5 Mol, vorzugsweise 1.0 bis 1.5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Oxidationsmittel zur Durchführung des erfindungsgemäßen Verfahrens (b) kann man alle üblicherweise für Schwefeloxidationen infrage kommenden anorganischen oder organischen Oxidationsmittel verwenden. Vorzugsweise verwendet man organische Persäuren, wie beispielsweise Peressigsäure, 4-Nitroperbenzoesäure oder 3-Chlorperbenzoesäure, anorganische Persäuren, wie beispielsweise Periodsäure oder auch Wasserstoffperoxid, Kaliumpermanganat oder Chromsäure.

Als Verdünnungsmittel zur Durchführung des erfindungsgemäßen Verfahrens (b) kommen ebenfalls inerte organische Lösungsmittel infrage.
Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (b) kann gegebenenfalls in Gegenwart eines Reaktionshilfsmittels, insbesondere eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (b) kann gegebenenfalls in Gegenwart eines geeigneten Katalysators durchgeführt werden. Als solche kommen alle üblicherweise für derartige Schwefeloxidationen üblichen Katalysatoren infrage. Beispielhaft genannt seien in diesem Zusammenhang Schwermetallkatalysatoren wie Ammoniummolybdat.

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +120 °C.

Zur Durchführung des erfindungsgemäßen Verfahrens (b) setzt man pro Mol an Triazoloazin der Formel (Ic) im allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an Triazoloazin der Formel (Ic) im allgemeinen 1,8 bis 3,0 Mol, vorzugsweise doppelt molare Mengen an Oxidationsmittel ein. Die Reaktionsführung, Aufarbeitung und Isolierung der Endprodukte der Formel (Ib) erfolgt nach üblichen Verfahren (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in monokotylen Kulturen wie beispielsweise Gerste oder Weisen einsetzen.

In geeigneten Aufwandmengen zeigen die erfindungsgemäßen Wirkstoffe außerdem eine fungizide Wirksamkeit und lassen sich beispielsweise zur Bekämpfung von Reiskrankheiten, wie beispielsweise gegen den Erreger der Reisfleckenkrankheit (Pyricularia oryzae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 4(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxybenzonitril (BROMOXYNIL); 2-Chlor-N-{[(4-methoxy-6-methyl-1,3,5-triazin-2-yl)-amino]-carbonyl}-benzolsulfonamid (CHLORSULFURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2- yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yl-oxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE) und 3-[[[[(4-Methoxy-6-methyl-1,3,5-triazin-2-yl)-amino]-carbonyl]-amino]-sulfonyl]-thiophen-2-carbonsäure-methylester (THIAMETURON) sind gegebenenfalls von Vorteil. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

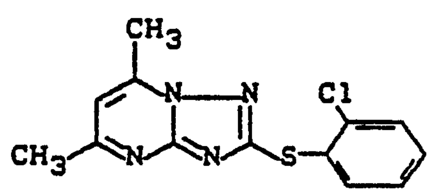

(Verfahren a)

Zu 4,85 g (0.0335 Mol) 2-Chlorthiophenol in 20 ml Sulfolan gibt man nacheinander 3,8 g (0.034 Mol) Kalium-t-butylat, 6,2 g (0.034 Mol) 2-Chlor-5,7-dimethyl-1,2,4-triazolo[1,5-a]pyrimidin (vgl. J. Chem. Soc. C 1966, 2031-2038) und erhitzt für 2 Minuten auf Rückflußtemperatur. Zur Aufarbeitung gießt man die

erkaltete Reaktionsmischung in Eiswasser, saugt den ausgefallenen Feststoff ab und kristallisiert aus wässrigem Ethanol um.

Man erhält 6,5 g (66,7 % der Theorie) an 2-(2-Chlorphenylthio)-5,7-dimethyl-1,2,4-triazolo[1,5-a]-pyrimidin vom Schmelzpunkt 143 °C.

Beispiel 2

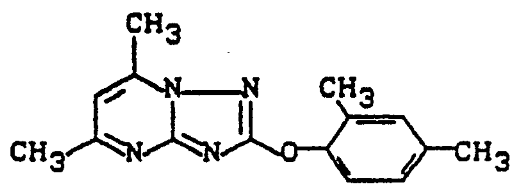

(Verfahren a)

Zu 1,5 g (0.0123 Mol) 2,4-Dimethylphenol in 15 ml Sulfolan gibt man nacheinander 1,35 g (0.0121 Mol) Kalium-t-butylat und 2.2 g (0.0121 Mol) 2-Chlor-5,7-dimethyl-1,2,4-triazolo[1,5-a]-pyrimidin und erhitzt für 15 Minuten auf Siedetemperatur. Zur Aufarbeitung gießt man die erkaltete Reaktionsmischung in Eiswasser, extrahiert mit Essigester, wäscht mit Wasser, trocknet über Natriumsulfat und entfernt das Lösungsmittel im Vakuum. Der Rückstand wird durch Chromatographie an Kieselgel gereinigt (Laufmittel: Dichlormethan/Aceton 4:1).

Man erhält 1,6 g (49,5 % der Theorie) an 2-(2,4-Dimethylphenoxy)-5,7-dimethyl-1,2,4-triazolo[1,5-a]-pyrimidin vom Schmelzpunkt 133-136 °C.

Beispiel 3

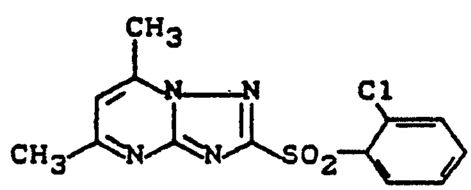

(Verfahren b)

Zu 3.0 g (0.01 Mol) 2-(2-Chlorphenylthio)-5,7-dimethyl-1,2,4-triazolo[1,5-a]pyrimidin in 100 ml Essigester gibt man 6,5 g (0.038 Mol) 3-Chlorperbenzoesäure und erhitzt für 2 Stunden auf Rückflußtemperatur. Zur Aufarbeitung wäscht man die erkaltete Reaktionsmischung mit wässriger Natriumcarbonatlösung, engt im Vakuum ein und kristallisiert den Rückstand aus Ethanol um.

Man erhält 1.8 g (54 % der Theorie) an 2-(2-Chlorphenylsulfonyl)-5,7-dimethyl-1,2,4-triazolo[1,5-a]-pyrimidin vom Schmelzpunkt 184 °C.

In entsprechender Weise und gemäß den allgemeinen Angaben zur Herstellung erhält man die folgenden Triazoloazine der allgemeinen Formel (I):

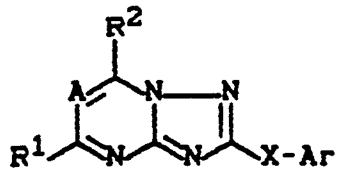

(I)

Tabelle 2

| Bsp. Nr. | $R^1$ | $R^2$ | A | X | Ar | Schmelzpunkt/°C |
|---|---|---|---|---|---|---|
| 4 | $CH_3$ | $CH_3$ | CH | S | 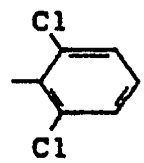 | 165-168 |
| 5 | $CH_3$ | $CH_3$ | CH | S | 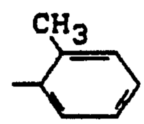 | 116-117 |
| 6 | $CH_3$ | $CH_3$ | CH | $SO_2$ | 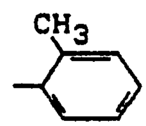 | 146-147 |
| 7 | $CH_3$ | $CH_3$ | CH | S | 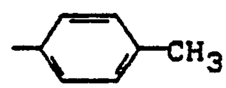 | 137-139 |
| 8 | $CH_3$ | $CH_3$ | CH | $SO_2$ | 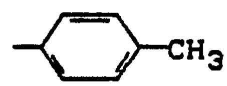 | 183-185 |
| 9 | $CH_3$ | $CH_3$ | CH | S | 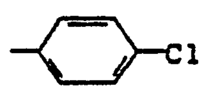 | 169-173 |
| 10 | $CH_3$ | $CH_3$ | CH | S | 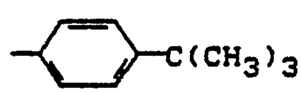 | 153-155 |
| 11 | $CH_3$ | $CH_3$ | CH | S | 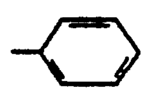 | 146-148 |
| 12 | $CH_3$ | $CH_3$ | CH | $SO_2$ | 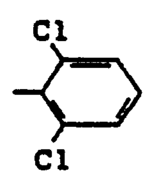 | 126-130 |

Tabelle 2 (Fortsetzung)

| Bsp. Nr. | $R^1$ | $R^2$ | A | X | Ar | Schmelzpunkt/° C |
|---|---|---|---|---|---|---|
| 13 | $CH_3$ | $CH_3$ | CH | $SO_2$ | 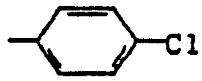 | 172-175 |
| 14 | $CH_3$ | $CH_3$ | CH | $SO_2$ | 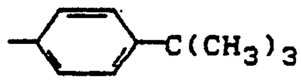 | 203-204 |
| 15 | $CH_3$ | $CH_3$ | CH | $SO_2$ | 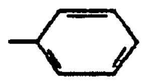 | 153-155 |
| 16 | $CH_3$ | $CH_3$ | CH | S | 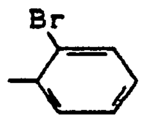 | 143 (Zers.) |
| 17 | $CH_3$ | $CH_3$ | CH | S | 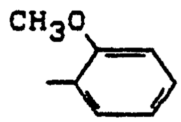 | 146 (Zers.) |
| 18 | $CH_3$ | $CH_3$ | CH | $SO_2$ | 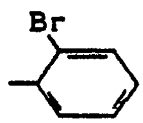 | 180-182 |
| 19 | $CH_3$ | $CH_3$ | CH | $SO_2$ | 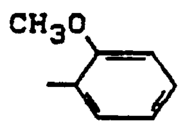 | 188-190 |
| 20 | $CH_3$ | $CH_3$ | CH | S | 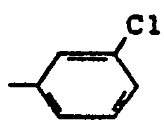 | 130 |
| 21 | $CH_3$ | $CH_3$ | CH | $SO_2$ | 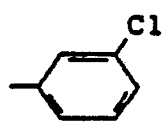 | 191-193 |

<u>Tabelle 2</u> (Fortsetzung)

| Bsp. Nr. | $R^1$ | $R^2$ | A | X | Ar | Schmelzpunkt/° C |
|---|---|---|---|---|---|---|
| 22 | $CH_3$ | $CH_3$ | CH | S | 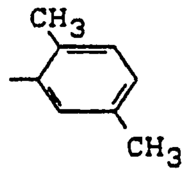 | 122-123 |
| 23 | $CH_3$ | $CH_3$ | CH | $SO_2$ | 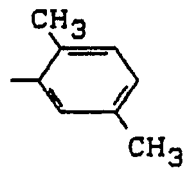 | 136-138 |
| 24 | $CH_3$ | $CH_3$ | CH | S | 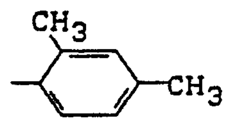 | 104-106 |
| 25 | $CH_3$ | $CH_3$ | CH | $SO_2$ | 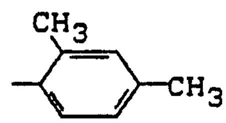 | 158-162 |
| 26 | $CH_3$ | $CH_3$ | CH | S | 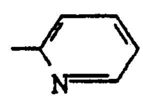 | 137 |
| 27 | $CH_3$ | $CH_3$ | C-Cl | S | 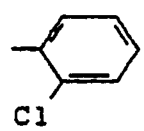 | |
| 28 | $CH_3$ | $CH_3$ | C-Cl | $SO_2$ | 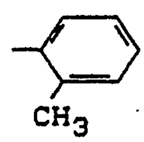 | |
| 29 | $CH_3$ | $CH_3$ | C-Cl | S | 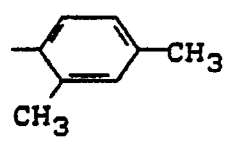 | |
| 30 | $CH_3$ | $CH_3$ | C-Cl | S | 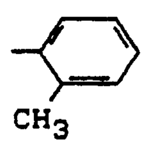 | |

Tabelle 2 (Fortsetzung)

| Bsp. Nr. | $R^1$ | $R^2$ | A | X | Ar | Schmelzpunkt/°C |
|---|---|---|---|---|---|---|
| 31 | $CH_3$ | $CH_3$ | C-Cl | $SO_2$ | 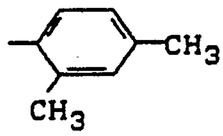 | |
| 32 | $CH_3$ | $CH_3$ | C-Cl | $SO_2$ | 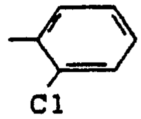 | |

## Anwendungsbeispiele

In dem folgenden Anwendungsbeispiel wird die nachstehend aufgeführte Verbindung als Vergleichsubstanz eingesetzt:

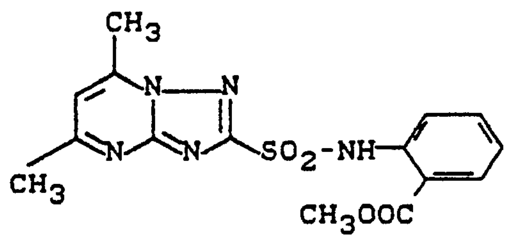

(A)

5,7-Dimethyl-N-(2-methoxycarbonyl-phenyl)-1,2,4-triazolo[1,5-a]pyrimidin-2-sulfonamid
(bekannt aus EP-A 142 152/Verbindung 10)

### Beispiel A

Post-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der herbiziden Wirksamkeit gegen dikotyle Unkräuter bei vergleichbarer Kulturpflanzenselektivität gegenüber der Vergleichsverbindung (A) zeigen in diesem Test z. B. die Verbindungen gemäß den Herstellungsbeispielen 24 und 25.

**Ansprüche**

1. Triazoloazine der allgemeinen Formel (I)

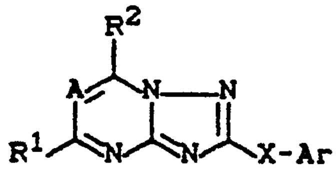

(I)

in welcher
A für Stickstoff oder für einen Rest C-$R^3$ steht,
$R^1$, $R^2$ und $R^3$ unabhängig voneinander jeweils für Wasserstoff, Hydroxy, Halogen, für gegebenenfalls substituiertes Alkyl, für Alkoxy, Alkylthio oder für gegebenenfalls substituiertes Aryl stehen oder entweder $R^1$ und $R^3$ gemeinsam oder $R^3$ und $R^2$ gemeinsam für einen ankondensierten gegebenenfalls substituierten Carbo-oder Heterocyclus stehen,
X für Sauerstoff, Schwefel, eine Sulfinylgruppe oder eine Sulfonylgruppe steht und
Ar für jeweils gegebenenfalls substituiertes Aryl oder Heteroaryl steht.

2. Triazoloazine der allgemeinen Formel (I) gemäß Anspruch 1, dadurch gekennzeichnet, daß darin
A für Stickstoff oder für einen Rest C-$R^3$ steht,
$R^1$, $R^2$ und $R^3$ unabhängig voneinander jeweils für Wasserstoff, Hydroxy, Fluor, Chlor, Brom, Iod, für jeweils geradkettiges oder verzweigtes Alkyl, Alkoxy oder Alkylthio mit jeweils 1 bis 6 Kohlenstoffatomen, für jeweils geradkettiges oder verzweigtes Alkoxyalkyl oder Alkylthioalkyl mit jeweils 1 bis 6 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen oder für gegebenenfalls einfach oder mehrfach, gleich oder verschieden substituiertes Aryl mit 6 bis 10 Kohlenstoffatomen stehen, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder jeweils geradkettiges oder verzweigtes Alkoxycarbonyl oder Alkoximinoalkyl mit jeweils 1 bis 4 Kohlenstoffatomen in den einzelnen Alkylteilen; oder entweder $R^1$ und $R^3$ gemeinsam oder $R^3$ und $R^2$ gemeinsam für einen ankondensierten gegebenenfalls einfach oder mehrfach, gleich oder verschieden substituierten, gesättigten oder ungesättigten Carbo- oder Heterocyclus stehen, wobei im Fall des Heterocyclus 1 bis 3 gleiche oder verschiedene Heteroatome enthalten sein können und wobei als Substituenten jeweils die oben genannten Arylsubstituenten infrage kommen,
X für Sauerstoff, Schwefel, eine Sulfinylgruppe oder eine Sulfonylgruppe steht und
Ar für gegebenenfalls einfach oder mehrfach, gleich oder verschieden substituiertes Aryl mit 6 bis 10 Kohlenstoffatomen oder für einen gegebenenfalls einfach oder mehrfach, gleich oder verschieden substituierten und/oder benzannellierten 5- bis 7-gliedrigen Heterocyclus mit 1 bis 3 Heteroatomen steht, wobei als Substituenten jeweils in Frage kommen: Fluor, Chlor, Brom, Iod, Cyano, Nitro, Hydroxy, jeweils geradkettiges oder verzweigtes Alkyl, Alkoxy, Alkylthio, Alkylcarbonyl, Alkylsulfinyl oder Alkylsulfonyl mit jeweils 1 bis 6 Kohlenstofatomen, jeweils geradkettiges oder verzweigtes Halogenalkyl, Halogenalkoxy, Halogenalkylthio, Halogenalkylsulfinyl, Halogenalkylsulfonyl oder Halogenalkylcarbonyl mit jeweils 1 bis 6 Kohlenstoffatomen und 1 bis 9 gleichen oder verschiedenen Halogenatomen, Phenyl, Phenoxy, Phenylthio, Phenylcarbonyl, Hydroxycarbonyl, jeweils geradkettiges oder verzweigtes Alkoxycarbonyl, Alkenyloxycarbonyl oder Alkoxyalkoxycarbonyl mit jeweils 1 bis 6 Kohlenstoffatomen in den einzelnen Alkylteilen bzw. 3 bis 6 Kohlenstoffatomen im Alkenylteil, sowie Hydroximinoalkyl oder geradkettiges oder verzweigtes Alkoximinoalkyl mit jeweils 1 bis 4 Kohlenstoffatomen in den einzelnen Alkylteilen.

3. Triazoloazine der allgemeinen Formel (I) gemäß Anspruch 1, dadurch gekennzeichnet, daß darin
A für Stickstoff oder für einen Rest C-$R^3$ steht,
$R^1$, $R^2$ und $R^3$ unabhängig voneinander jeweils für Wasserstoff, Hydroxy, Fluor, Chlor, Brom, für Methyl, Ethyl, n- oder i-Propyl, n-, i-, s- oder t-Butyl, für Chlormethyl, Dichlormethyl, Trichlormethyl, Fluormethyl, Difluormethyl, Trifluormethyl, Dichlorfluormethyl, Chlordifluormethyl oder Fluorchlormethyl, für Methoxymethyl, Ethoxymethyl, n- oder i-Propoxymethyl, Methylthiomethyl, Ethylthiomethyl, n- oder i-Propylthiomethyl, für Methoxy, Ethoxy, n- oder i-Propoxy, für Methylthio, Ethylthio, n- oder i-Propylthio oder für Phenyl stehen,
X für Sauerstoff, Schwefel, eine Sulfinylgruppe oder eine Sulfonylgruppe steht und

Ar für jeweils gegebenenfalls ein- bis dreifach, gleich oder verschieden substituiertes Phenyl oder Naphthyl oder für einen gegebenenfalls ein- bis dreifach gleich oder verschieden substituierten und/oder benzannellierten Heterocyclus der Formel

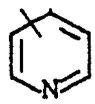 ; 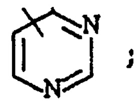 ; 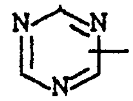 ; 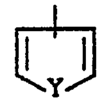 ; 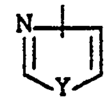 ;

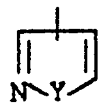 ; 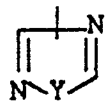 oder 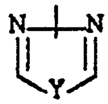 steht,

wobei Y jeweils für Sauerstoff, Schwefel, eine NH-Gruppe oder eine N-$CH_3$-Gruppe steht und wobei als Phenyl-, Naphthyl- und Heterocyclyl-Substituenten jeweils in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, n- oder i-Propoxy, Methylthio, Ethylthio, Acetyl, Propionyl, Methylsulfinyl, Methylsulfonyl, Trifluormethyl, Trifluormethoxy, Trifluormethylthio, Trifluormethylsulfinyl, Trifluormethylsulfonyl, Chloracetyl, Dichloracetyl, Trifluoracetyl, Phenyl, Phenoxy, Phenylthio, Benzoyl, Hydroxycarbonyl, Methoxycarbonyl, Ethoxycarbonyl, n- oder i-Propoxycarbonyl, Allyloxycarbonyl, Methoxymethoxycarbonyl, Ethoxyethoxycarbonyl, Hydroximinomethyl, Methoximinomethyl, Methoximinoethyl und Ethoximinoethyl.

4. Triazoloazine der allgemeinen Formel (I) gemäß Anspruch 1, dadurch gekennzeichnet, daß darin
A für Stickstoff oder für eine CH-Gruppe steht,
$R^1$ und $R^2$ unabhängig voneinander für Wasserstoff, für Methyl, Ethyl, n- oder i-Propyl, n-, i-, s-oder t-Butyl, für Methoxymethyl, Ethoxymethyl, n- oder i-Propoxymethyl, für Methylthiomethyl, für Trifluormethyl, Trichlormethyl, für Dichlorfluormethyl, Chlordifluormethyl, für Methoxy, Ethoxy, n- oder i-Propoxy, Methylthio oder Ethylthio stehen,
X für Sauerstoff, Schwefel, eine Sulfinylgruppe oder eine Sulfonylgruppe steht und
Ar für jeweils gegebenenfalls ein- bis dreifach, gleich oder verschieden substituiertes Phenyl, α-Naphthyl oder β-Naphthyl steht oder für einen gegebenenfalls ein- bis dreifach gleich oder verschieden substituierten und/oder benzannellierten Heterocyclus der Formel

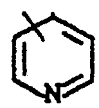 ; 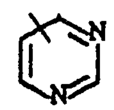 ; 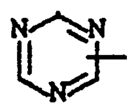 ; 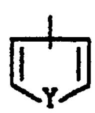 ; 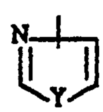 oder

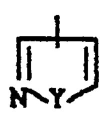 steht,

wobei Y jeweils für Sauerstoff, Schwefel, eine NH-Gruppe oder eine N-$CH_3$-Gruppe steht und wobei als Phenyl-, Naphthyl- und Heterocyclyl-Substituenten jeweils infrage kommen: Fluor, Chlor, Brom, Iod, Cyano, Nitro, Hydroxy, Methyl, Ethyl, Methoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

5. Triazoloazine der allgemeinen Formel (I) gemäß Anspruch 1, dadurch gekennzeichnet, daß darin
A für eine CH-Gruppe steht,
$R^1$ für Methyl, Methoxy, Methoxymethyl oder Trifluormethyl steht,
$R^2$ für Wasserstoff, Methyl, Methoxy, Methoxymethyl oder Trifluormethyl steht,
X für Sauerstoff, Schwefel oder eine Sulfonylgruppe steht und
Ar für jeweils gegebenenfalls ein- bis dreifach, gleich oder verschieden substituiertes Phenyl, Pyridyl oder Pyrimidyl steht, wobei als Substituenten jeweils in Frage kommen: Fluor, Chlor, Brom, Cyano, Nitro, Hydroxy, Methyl, Ethyl, t-Butyl, Methoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

6. Verfahren zur Herstellung von Triazoloazinen der Formel (I )

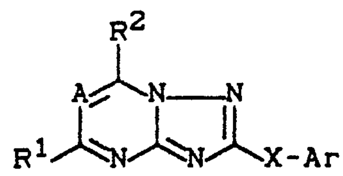

(I)

in welcher
A für Stickstoff oder für einen Rest C-$R^3$ steht,
$R^1$, $R^2$ und $R^3$ unabhängig voneinander jeweils für Wasserstoff, Hydroxy, Halogen, für gegebenenfalls substituiertes Alkyl, für Alkoxy, Alkylthio oder für gegebenenfalls substituiertes Aryl stehen oder entweder $R^1$ und $R^3$ gemeinsam oder $R^3$ und $R^2$ gemeinsam für einen ankondensierten gegebenenfalls substituierten Carbo-oder Heterocyclus stehen,
X für Sauerstoff, Schwefel, eine Sulfinylgruppe oder eine Sulfonylgruppe steht und
Ar für jeweils gegebenenfalls substituiertes Aryl oder Heteroaryl steht,
dadurch gekennzeichnet daß man Triazoloazine der Formel (Ia)

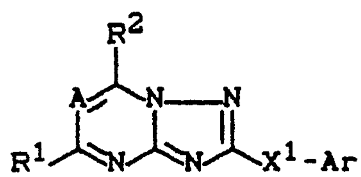

(Ia)

in welcher
$X^1$ für Sauerstoff oder Schwefel steht und
$R^1$, $R^2$, A und Ar die oben angegebene Bedeutung haben,
erhält, wenn man Triazoloazin-Derivate der Formel (II),

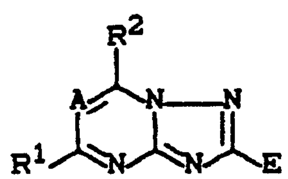

(II)

in welcher
E für Halogen, für Alkylthio oder für Alkylsulfonyl steht und
$R^1$, $R^2$ und A die oben angegebene Bedeutung haben,
mit aromatischen Alkoholen oder Thiolen der Formel (III),
Ar-$X^1$-H (III)
in welcher
$X^1$ und Ar die oben angegebene Bedeutung haben,
gegebenenfalls in Gegenwart eines Verdünnungsmittels und gegebenenfalls in Gegenwart eines Reaktionshilfsmittels umsetzt, oder daß man
Triazoloazine der Formel (Ib)

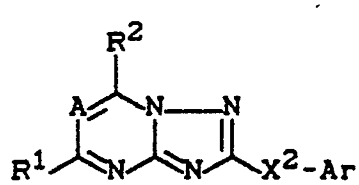

(Ib)

in welcher
$X^2$ für eine Sulfinylgruppe oder eine Sulfonylgruppe steht und
$R^1$, $R^2$, A und Ar die oben angegebene Bedeutung haben,
erhält, wenn man Triazoloazine der Formel (Ic),

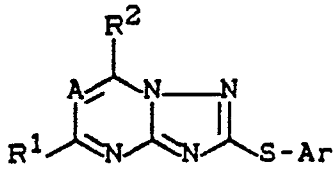

(Ic)

in welcher
$R^1$, $R^2$, A und Ar die oben angegebene Bedeutung haben,
mit einem Oxidationsmittel gegebenenfalls in Gegenwart eines Verdünnungsmittels und gegebenenfalls in Gegenwart eines Katalysators umsetzt.

7. Herbizide Mittel, gekennzeichnet durch einen Gehalt an mindestens einem Triazoloazin der Formel (I) gemäß den Ansprüchen 1 bis 6.

8. Verfahren zur Bekämpfung von Unkräutern, dadurch gekennzeichnet, daß man Triazoloazine der Formel (I) gemäß den Ansprüchen 1 bis 6 auf die Unkräuter und/oder ihren Lebensraum einwirken läßt.

9. Verwendung von Triazoloazinen der Formel (I) gemäß den Ansprüchen 1 bis 6 zur Bekämpfung von Unkräutern.

10. Verfahren zur Herstellung von herbiziden Mitteln, dadurch gekennzeichnet, daß man Triazoloazine der Formel (I) gemäß den Ansprüchen 1 bis 6 mit Streckmitteln und/oder oberflächenaktiven Substanzen vermischt.

Europäisches Patentamt

# EUROPÄISCHER RECHERCHENBERICHT

Nummer der Anmeldung

EP 89 10 5800

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 256 396 (BAYER AG)<br>* Ansprüche 1,5-8,10 *<br>--- | 1,7-10 | C 07 D 487/04<br>A 01 N 43/90 //<br>(C 07 D 487/04<br>C 07 D 251:00<br>C 07 D 249:00<br>C 07 D 487:04<br>C 07 D 249:00<br>C 07 D 239:00 ) |
| A | EP-A-0 244 847 (DOW CHEMICAL CO.)<br>* Anspruch 1; Seite 14, Zeilen 1-14 *<br>--- | 1,7-10 | |
| D,A | EP-A-0 142 152 (DOW CHEMICAL CO.)<br>* Ansprüche 1,43,44 *<br>--- | 1,7-9 | |
| A | EP-A-0 150 974 (DOW CHEMICAL CO.)<br>* Ansprüche 1,7,8 *<br>--- | 1,7-9 | |
| A | US-A-4 685 958 (N. R. PEARSON et al.)<br>* Ansprüche 1,4,7 *<br>--- | 1,7-9 | |
| A | US-A-4 605 433 (N. R. PEARSON et al.)<br>* Ansprüche 1,4,10,17,18 *<br>--- | 1,7-9 | |
| D,A | US-A-4 036 840 (D. E. O BRIEN et al.)<br>* Tabelle IV *<br>--- | 6 | **RECHERCHIERTE SACHGEBIETE (Int. Cl.4)** |
| D,A | EP-A-0 197 895 (CIBA-GEIGY AG)<br>* Anspruch 1 *<br>----- | 6 | C 07 D 487/00 |

Der vorliegende Recherchenbericht wurde für alle Patentansprüche erstellt

| Recherchenort | Abschlußdatum der Recherche | Prüfer |
|---|---|---|
| BERLIN | 05-07-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)